# EUROPÄISCHE PATENTSCHRIFT

(11) **EP 0 994 172 B1**
(45) Veröffentlichungstag und Bekanntmachung des Hinweises auf die Patenterteilung: **17.11.2004**
(21) Anmeldenummer: 99117800.5
(22) Anmeldetag: 09.09.1999
(51) Int. Cl.: C09J 183/00, A61K 6/093, C08F 8/42

(54) **Einkomponentenadhäsiv**
Single-component adhesive
Adhésif à un composant

(30) Priorität: 13.10.1998 DE 19847116; 29.10.1998 DE 19849966
(43) Veröffentlichungstag der Anmeldung: 19.04.2000
(73) Patentinhaber: Ernst Mühlbauer GmbH & Co.KG, 25870 Norderfriedrichskoog (DE)
(72) Erfinder: Neffgen, Stephan, Dr., 22459 Hamburg (DE)
(74) Vertreter: Glawe, Delfs, Moll & Partner

(56) Entgegenhaltungen:
- EP-A- 0 632 060
- EP-A- 0 685 547
- EP-A- 0 826 359

## Beschreibung

Gegenstand der Erfindung ist ein polymeres Adhäsiv gemäß dem Hauptanspruch, Verfahren zu dessen Herstellung sowie dessen Verwendung als medizinisches Präparat und zur Herstellung eines Haftvermittlers zur Anwendung im Dentalbereich.

Zahnprothesen bestehen in der Regel aus einem harten Kunststoff, häufig wird Polymethylmethacrylat (PMMA) verwendet. Zur Verbesserung der Paßgenauigkeit und zur Vermeidung von Druckstellen auf der Mundschleimhaut kann es erforderlich sein, solche Zahnprothesen mit einem dauerhaft weichbleibenden Unterfütterungsmaterial zu versehen. Als Unterfütterungsmaterialien werden auch Organopolysiloxane (Silikone) verwendet. Es ist jedoch problematisch, eine dauerhafte Verbindung zwischen dem Prothesenkunststoff und den Silikonen als Unterfütterungsmaterial herzustellen.

DE-A-44 14 837 beschreibt einen Haftvermittler zur Herstellung einer Verbindung zwischen PMMA und einem siliciumorganischen Elastomer. Auf den Prothesenkunststoff wird zunächst ein Zweikomponenten-Polyurethan-Reaktionsklebstoff aufgetragen, der nach einer gewissen Aushärtungszeit mit einem Organohydrogen-Silikonölfilm versehen wird. Auf diesen Film wird das Unterfütterungsmaterial aufgebracht. Die Applikation eines solchen Mehrkomponenten-Haftvermittlers ist kompliziert und langwierig.

Einkomponenten-Adhäsive sind offenbart in EP-A-0 632 060 und EP-A-0 826 359. Es hat sich jedoch gezeigt, daß die dort offenbarten Adhäsive hydrolyseempfindlich und wenig stabil sind. Haftvermittlersysteme sind ferner aus DE-A-195 39 653, EP-A-0 685 547, EP-A-0 384 401, EP-A-0 731 143 und US-A-5,635,578 bekannt.

Der vorliegenden Erfindung liegt die Aufgabe zugrunde, ein Adhäsiv der eingangs genannten Art zu schaffen, das eine gute Haftung zwischen einem Kunststoff und einem Organosilikonmaterial vermitteln kann, einfach und schnell anwendbar sowie chemisch stabil ist.

Gelöst wird diese Aufgabe erfindungsgemäß durch ein Adhäsiv, das ein Polymer mit den folgenden Merkmalen enthält:
- ein polymerer Backbone,
- über Esterbindungen mit dem Backbone verknüpfte Alkyl-, Aryl- und/oder Alkenylgruppen
- über Esterbindungen mit dem Backbone verknüpfte Alkylen- und/oder Arylengruppen als Spacer,
- an den Spacer ist mittels einer Si-C-Bindung eine offenkettige und/oder cyclische Silikongruppe und/oder Silangruppe gebunden,
- an die Silikon- bzw. Silan-Gruppe ist mittels einer Si-C-Bindung eine Ethylengruppe gebunden,
- die Ethylengruppe wiederum ist mit einer zweiten Silikongruppe verbunden, die M-, D-, T-, und/oder Q-Silikoneinheiten sowie wenigstens eine an Si gebundene Vinylgruppe enthält.

Das erfindungsgemäße Adhäsiv enthält ein polymeres Grundgerüst (Backbone). Es kann sich bspw. um ein Poly(alk)acrylat handeln. Diese Terminologie schließt Polyacrylate und Polyalkacrylate wie bspw. Polymethacrylat ein.

Über Esterbindungen sind mit dem Backbone Alkyl- und/oder Arylgruppen (diese Terminologie schließt Aralkylgruppen ein) als Alkoholkomponenten verbunden. Die genannten Alkylund/oder Arylgruppen können zusätzliche funktionelle Gruppen wie bspw. Hydroxygruppen tragen. Die genannten Alkoholkomponenten sind bevorzugt Ester von Methanol, Ethanol, Propanol, Isopropanol, Butanol, tert.-Butanol, Pentanol, Hexanol, Cyclohexanol, Octanol, Decanol, Laurylalkohol, Cetylalkohol, Propinalkohol, Glycerol, Diethylenglycol, Triethylenglycol, Benzylalkohol und dessen Derivate, Phenol und dessen Derivate, 2-Hydroxyethoxybenzol sowie 2-Hydroxyethylbenzol, die Esterbindung erfolgt mit einer Säure- oder Säurederivatfunktion am Backbone.

Ebenfalls über Esterbindungen mit dem Backbone verknüpft sind als Spacer dienende verzweigte oder unverzweigte Alkylen- und/oder Arylengruppen. Bevorzugt werden als Spacer geradkettige unverzweigte Alkylketten mit einer Kettenlänge von C₃ bis C₁₈, besonderes bevorzugt C₃ sowie C₆ bis C₁₈ verwendet. Der Spacer kann funktionelle Gruppen wie bspw. Estergruppen, Etherfunktionen oder zusätzliche Doppelbindungen enthalten.

An den Spacer ist mittels einer stabilen Si-C-Bindung eine offenkettige und/oder cyclische Silikongruppe (ein Organosiloxan) und/oder eine Silangruppe gebunden. Der Begriff Silikongruppe schließt jede Gruppe ein, die wenigstens eine Siloxanbindung (Si-O-Si-Bindung) enthält.

An diesen verlängerten Spacer ist ebenfalls mittels einer stabilen Si-C-Bindung eine Ethylengruppe gebunden, die wiederum mit einer zweiten Silikongruppe verbunden ist, die M-, D-, T- und/oder Q-Silikoneinheiten sowie wenigstens eine an ein Si-Atom gebundene ethylenisch ungesättigte Gruppe (Vinylgruppe) enthält. Die Terminologie M, D, T und Q zur Bezeichnung von Silikoneinheiten ist international üblich (siehe bspw. Römpp Chemielexikon, 9. Aufl., Bd. 9, S. 4168) und bezeichnet Silikoneinheiten mit den folgenden Strukturen:

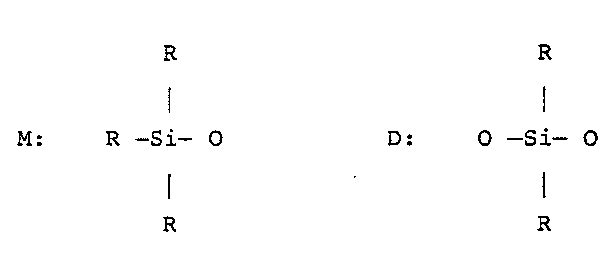

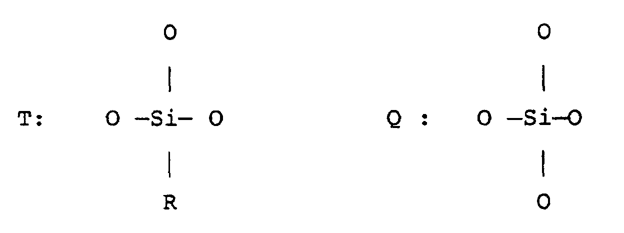

Der Rest R bezeichnet Alkyl-, Aryl- oder Aralkylgruppen.

Erfindungsgemäß enthält die genannte zweite Silikongruppe wenigstens eine an Si gebundende ethylenisch ungesättigte Gruppe. Mittels dieser an der zweiten Silikongruppe angeordneten reaktiven Doppelbindung kann das Adhäsiv eine chemische Bindung zu dem siliciumorganischen Elastomer herstellen.

Das erfindungsgemäße Adhäsiv weist zwei wesentliche Vorteile gegenüber dem Stand der Technik auf. Zum einen enthält das Adhäsiv als reaktive Gruppe in den Silikoneinheiten ethylenisch ungesättigte Doppelbindungen und keine Si-H-Bindungen. Da die Si-H-Bindung mit Nucleophilen reagieren kann, ist das erfindungsgemäße Adhäsiv wesentlich stabiler (insbesondere lagerstabiler).

Die Verknüpfung zwischen dem Backbone und den Silikongruppen erfolgt ausschließlich über stabile Si-C-Bindungen, nicht über hydrolyseempfindliche Si-O-C-Bindungen.

Die unmittelbar mit dem Spacer verknüpfte offenkettige und/oder cyclische Silikongruppe weist bevorzugt eine Silaneinheit bzw. 1 bis 10, besonders bevorzugt 1 bis 5 Silikoneinheiten (Si-O-Einheiten) auf, die linear, verzweigt und/oder cyclisch angeordnet sein können. Alkyl-, Arylund/oder Aralkylseitengruppen können vorhanden sein, bevorzugt sind Methyl-, Ethyl-, Phenyl- und Benzylgruppen.

Das Adhäsiv weist bevorzugt folgende Strukturelemente auf: 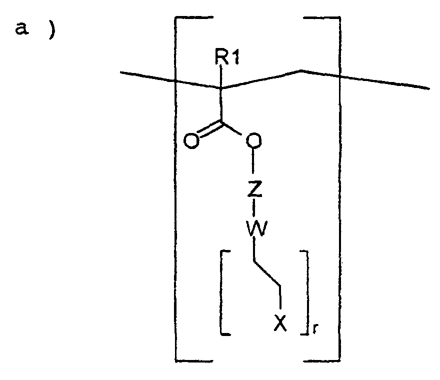 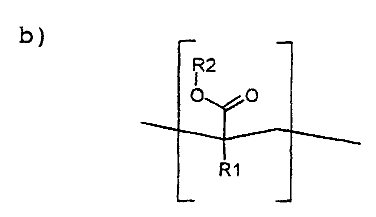 wobei bedeuten:
- r: eine ganze Zahl von 1 bis 3,
- R₁: gleich oder verschieden H, Methyl, Ethyl, Propyl oder Butyl,
- R₂: eine geradkettige oder verzweigte Alkyl-, Aryl-, Aralkyl- und/oder Alkenylgruppe,
- Z: eine geradkettige oder verzweigte Alkyl- oder Aryl- oder Aralkylgruppe,
- W: eine offenkettige und/oder cyclische Silikongruppe oder Silangruppe, die über Si-C-Bindungen verknüpft ist,
- X: eine M-, D-, T- und/oder Q-Einheiten enthaltende zweite Silikongruppe, wobei wenigstens eine Silikongruppe X wenigstens eine an Si gebundene Vinylgruppe enthält.

Die genannten Strukturelemente a) und b) können alternierend oder regellos im Backbone angeordnet sein. Wie aus der Formel a) hervorgeht, kann die Silikongruppe W zwischen 1 und 3 über eine Ethylengruppe angeknüpfte zweite Silikongruppe(n) mit wenigstens einer Vinylgruppe enthalten.

Das Strukturelement a) kann wie folgt aufgebaut sein: 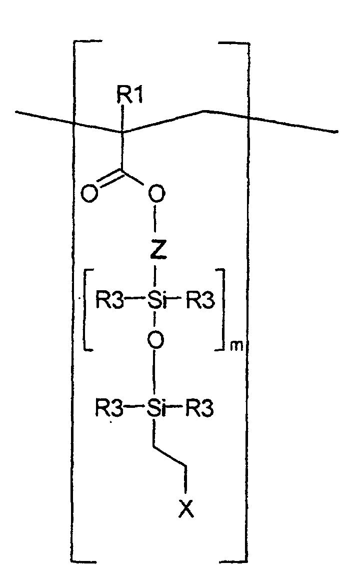 wobei R₁, R₂, Z und X die in Anspruch 2 angegebenen Bedeutungen haben, R₃ eine geradkettige oder verzweigte Alkyl- oder Aryl- oder Aralkylgruppe darstellt und m eine ganze Zahl von 0 bis 2 ist.

Das Grundgerüst kann ein Poly(alk)acrylat, insbesondere Poly(meth)acrylat, aufweisen. Wenn im Rahmen der Erfindung der Ausdruck Acrylat bzw. Polyacrylat verwendet wird, schließt dies immer auch Polyalkacrylate ein, die am α-ständigen C-Atom eine Alkylgruppe, wie bspw. eine Methylgruppe, tragen. Die Poly(alk)acrylatkomponente kann ein Molekulargewicht zwischen 800 und 1.000.000 aufweisen. Bevorzugt sind Molmassen zwischen 5.000 und 500.000, besonderes bevorzugt zwischen 20.000 und 250.000.

Statt eines reinen Poly(alk)acrylates kann der Backbone auch ein Copolymer von (Alkyl)acrylaten und anderen ungesättigten Estern sein.

Die zur Verbindung mit dem siliciumorganischen Elastomer vorgesehene Silikongruppe (X in Anspruch 2) weist bevorzugt ein Molekulargewicht zwischen 80 und 200.000 auf. Wenn an der offenkettigen oder cyclischen Silikoneinheit (W in Anspruch 2) mehrere Silikongruppen X angebunden sind, bezieht sich die genannte Molmassenangabe auf eine dieser Silikongruppen. Nicht jede als Seitenkette der Silikongruppe W ausgebildete Silikongruppe X muß notwendiger Weise eine reaktive Vinylgruppe aufweisen, mindestens eine Silikongruppe X weist jedoch mindestens eine reaktive Vinylgruppe auf.

Der Spacer (Z in Anspruch 2) ist bevorzugt eine geradkettige, d.h. unverzweigte Alkylverbindung.

Das erfindungsgemäße Adhäsiv kann zusätzlich ein nichtreaktives Lösemittel enthalten. "Nichtreaktiv" bedeutet, daß das Lösemittel unter den Lagerungs- und Anwendungsbedingungen des Adhäsivs sich im wesentlichen chemisch inert verhält. Geeignete Lösemittel sind bspw. Ethylacetat, Toluol, Benzol, Methylenchlorid, Chloroform, Isobutanol, Isopropanol, Ethanol, Methanol, Cyclohexanol, Cycolhexanon, Aceton, Tetrahydroforan, Cyclohexylacetat, Essigsäure, Methylethylketon, Diethylether oder Mischungen daraus.

Das Adhäsiv kann zusätzlich reaktive (Meth)acrylatharze enthalten. Wie oben bereits erläutert, schließt diese Terminologie Acrylatharze und Methacrylatharze ein. Geeignete (Meth)acrylatharze sind bspw. Ethylenglycoldi(meth)acrylat, Diethylenglycoldi(meth)acrylat, Triethylenglycoldi(meth)acrylat, Tetraethylenglycoldi(meth)-acrylat, Hexandioldi(meth)acrylat, Butandioldi(meth)-acrylat, Dodecandioldi(meth)acrylat, 2,2-Bis[p-(hydroxy(meth)acryloyloxy)phenyl]propan, ethoxyliertes Bisphenol-A-di(meth)acrylat, Hydroxyethyl(meth)acrylat, Hydroxypropyl(meth)acrylat, Glycerindi(meth)acrylat, Urethandi(meth)acrylat, Urethanpolyesterdi(meth)acrylat, Trimethylolpropantri(meth)acrylat und Dipentaerythritpenta(meth)acrylat.

Das Adhäsiv kann zusätzlich amorphe Kieselsäuren, Silikate, feinteilige Gläser und/oder Quarze und/oder Pigmente zur Einfärbung enthalten. Beispielhaft seien silanisierte Aerosile genannt.

Gegenstand der Erfindung ist ferner ein Verfahren zur Herstellung eines solchen Adhäsivs mit den Schritten:
- Umesterung eines Poly(alk)acrylats mit ethylenisch ungesättigten Alkoholen,
- Umsetzung dieser Verbindung mit flüchtigen Silanen und/oder Siloxanen, die mindestens eine und wenigstens teilweise mindestens zwei Si-H-Bindungen enthalten,
- Umsetzung des Reaktionsproduktes mit Silikonen, die mindestens eine an Si gebundene Vinylgruppe und wenigstens teilweise zwei oder mehr an Si gebundene Vinylgruppen enthalten.

Im ersten Schritt der Herstellung wird ein polymeres Acrylat oder Alkacrylat (bspw. Methacrylat) mit ethylenisch ungesättigten Alkoholen umgesetzt. Benutzt man die Terminologie des Anspruchs 2, wird in diesem Schritt die per Esterbindung an den Backbone gebundene Gruppe R₂ durch einen Spacer Z ersetzt. Es erfolgt vorzugsweise keine vollständige Umesterung aller Estergruppen am Backbone, im Polymer verbleibt somit vorzugsweise ein gewisser Anteil von Estergruppen R₂.

Im nächsten Schritt wird die erhaltene Verbindung mit flüchtigen Silanen bzw. Siloxanen mit mindestens 2 Si-H-Bindungen umgesetzt. Dabei reagiert eine Si-H-Bindung mit der ethylenisch ungesättigten Gruppe des Spacers Z, so daß die Silikongruppe W an den Spacer Z angeknüpft wird. Diese Reaktion kann so durchgeführt werden, daß die ethylenisch ungesättigten Estergruppen fast vollständig mit den Silanen reagieren, alternativ können jedoch auch ethylenisch ungesättigte Ester im Polymer verbleiben.

Im dritten Schritt wird das Reaktionsprodukt mit Silikonen umgesetzt, die wenigstens teilweise zwei oder mehr an Si gebundene ethylenisch ungesättigte Gruppen enthalten. Die noch vorhandene Si-H-Bindung der ersten Silikongruppe W reagiert dabei mit einer Vinylgruppe der anzuknüpfenden zweiten Silikongruppe X (jeweils in der Terminologie des Anspruchs 2). Wenn das im zweiten Reaktionsschritt angeknüpfte flüchtige Silan mehr als zwei Si-H-Bindungen aufweist, können u.U. mehrere Silikongruppen X durch Addition einer Si-H-Bindung an eine Vinylgruppe angeknüpft werden. In diesem Fall ist r in Anspruch 2 größer als 1. Die im letzten Reaktionsschritt angebundenen zweiten Silikongruppen enthalten jeweils mindestens zum Teil noch eine freie Vinylgruppe, die der Bindung des Adhäsivs an ein siliciumorganisches Elastomer dient.

Das erfindungsgemäße hergestellte Adhäsiv kann dann folgende Struktur aufweisen:

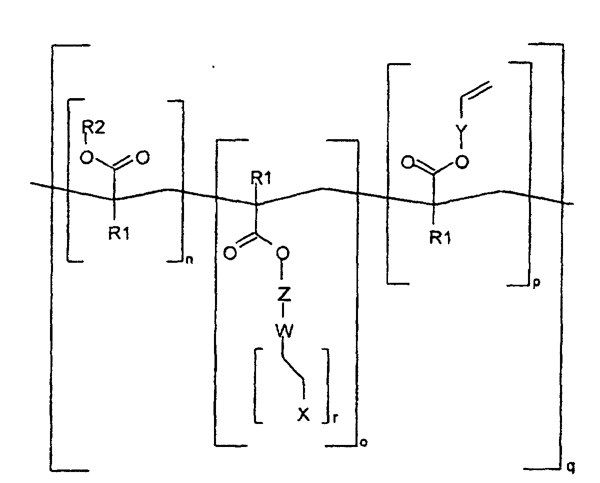

Die Formeleinheit n stellt ein ursprüngliches Poly(alk)acrylat dar, das noch die ursprüngliche Estergruppe trägt. Die Formeleinheit p zeigt einen Polyacrylatbaustein, mit dem über den Ester eine Alkenylgruppe verknüpft ist.Y bedeutet hier eine geradkettige oder verzweigte Alkyl- oder Aryl- oder Aralkylgruppe. Bei der Einheit o ist dagegen über die Gruppe Z eine Silan- bzw. Siloxangruppe angeknüpft, die über die Gruppe W mindestens eine Silikongruppe X aufweist. Wenigstens eine Silikongruppe X weist mindestens eine an Si gebundene Vinylgruppe auf. In einem erfindungsgemäßen Adhäsiv muß eine Formeleinheit p nicht notwendigerweise enthalten sein. Die Abfolge der Formeleinheiten n und o innerhalb einer Einheit q ist beliebig und kann unterschiedlich sein, jedoch müssen in dem Adhäsiv sowohl Einheiten n als auch Einheiten o vorhanden sein.

Ein zweites erfindungsgemäßes Verfahren zur Herstellung eines erfindungsgemäßen Adhäsivs weist folgende Schritte auf:
- Umsetzung eines Copolymeren aus Alkyl(meth)acrylaten und Alkenyl(meth)acrylaten mit flüchtigen Silanen und/oder Siloxanen, die mindestens eine und wenigstens teilweise mindestens zwei Si-H-Bindungen enthalten,
- Umsetzung des Reaktionsproduktes mit Silikonen, die mindestens eine an Si gebundene Vinylgruppe und wenigstens teilweise zwei oder mehr an Si gebundene Vinylgruppen enthalten.

Als Startpolymer setzt man hier ein Copolymer aus Alkyl(meth)acrylaten und Alkenyl(meth)acrylaten ein, das von vornherein reaktive Doppelbindungen enthält, an die Si-H-Bindungen von Silanen addiert werden können. Bei Einsatz eines solchen Copolymers ist es nicht erforderlich, das Polymer zunächst durch Umesterung ethylenisch ungesättigten Alkoholen mit solchen reaktiven Doppelbindungen zu versehen. An die reaktiven Doppelbindungen des Copolymers werden in einem ersten Schritt flüchtige Silane mit mindestens zwei Si-H-Bindungen addiert. Dieses Reaktionsprodukt wird in einem zweiten Schritt mit Silikonen umgesetzt, die mindestens zwei an Si gebundene Vinylgruppen enthalten. Die beiden Reaktionsschritte entsprechend dem zweiten und dritten Reaktionsschritt des obengenannten ersten erfindungsgemäßen Verfahrens.

Die Erfindung betrifft ferner ein in den Ansprüchen definiertes Adhäsiv zur Verwendung als medizinisches Präparat sowie die Verwendung dieses Adhäsivs zur Herstellung eines Haftvermittlers zur Anwendung im Dentalbereich zwecks Herstellung einer kraftschlüssigen Verbindung zwischen einem Kunststoff und einem Organosiloxanmaterial.

Das erfindungsgemäße Adhäsiv kann direkt auf einen Kunststoff aufgetragen werden. Sofern es ein Lösemittel enthält, läßt man dieses verdampfen. Auf die Adhäsivschicht wird anschließend ein siliciumorganisches Elastomer aufgebracht.

Ein erfindungsgemäß hergestellter Haftvermittler kann bspw. zur Verbindung zwischen einer Dentalprothese aus einem harten Kunststoff und einem siliciumorganischen Unterfütterungsmaterial dienen. Eine weitere Anwendungsmöglichkeit ist die Haftvermittlung zwischen einem individuellen Abformlöffel aus Kunststoff und einem Abformmaterial.

Ebenso ist dieses Adhäsiv im otoplastischen verwendbar, bspw. für die Verbindung von Ohrschalen mit einer weicheren Silikonauflage oder auch zur kraftschlüssigen Verbindung mit anderen Silikonbauteilen wie bspw. Schläuchen zu verwenden. Bei den Ohrschalen kann es sich bspw. um Hörgerätebauteile aus einem harten Kunststoff handeln.

Ausführungsbeispiele der Erfindung werden nachfolgend beschrieben. Sofern nicht anders spezifiziert, sind alle Zusammensetzungen in Gewichtsanteilen angegeben. Viskositäten sind bei einer Temperatur von 25°C gemessen worden.

### Materialien

Die im folgenden beschriebenen Edukte wurden bei der Herstellung der Haftvermittlersysteme eingesetzt:

**Tabelle 1**

| | |
|---|---|
| PA-30 | Poly(methylmethacrylat) mit kleineren Anteilen Acrylat-Comonomer; Molmasse ≈ 30.000 |
| PA-180 | Poly(methylmethacrylat) mit kleineren Anteilen Acrylat-Comonomer; Molmasse ≈ 180.000 |
| DVPDMS | Lineares α,ω-Divinylpoly(dimethylsiloxan); η ≈ 25 mPa·s; Vinylgehalt ≈ 1 mmol g⁻¹ |
| VMQS | Verzweigtes vinylhaltiges Poly(siloxan); η ≈ 2000 mPa·s; Vinylgehalt ≈ 0,8 mmol g⁻¹ |
| VMDTS | Verzweigtes vinylhaltiges Poly(siloxan); η ≈ 490 mPa s; Vinylgehalt ≈ 1,1 mmol g⁻¹ |
| TMDS | 1,1,3,3-Tetramethyldisiloxan |
| Pt-Katalysator | Pt⁰-Komplex mit 1,3-Divinyl-1,1,3,3-tetramethyldisiloxan; 10,7%ige Lösung in Toluol |

### Beispiel 1:

### Herstellung eines Umesterungskatalysators

2 Teile Titantetraisopropanolat werden in 5,5 Teilen getrockneten 9-Decen-1-ols unter Feuchtigkeitsausschluß gelöst. Anschließend werden 0,05 Teile 2,6-Ditertiärbutyl-4-methylphenol zugegeben und der Ansatz bei 70°C und ca. 20 mbar 4 h unter Rühren umgesetzt. Das Produkt wird ohne weitere Reinigung in den nächsten Stufen eigesetzt.

### Beispiel 2:

### Herstellung eines vinylmodifizierten Polymethylmethacrylats (VPA 1)

100 Teile trockenes Polymethylmethacrylat PA-180 (Copolymer mit geringeren Anteilen anderer Acrylate) werden in ca. 350 Teilen trockenem Toluol unter Feuchtigkeitsausschluß gelöst und diese Lösung mit 0,03-0,04 Teilen 2,6-Ditertiärbutyl-4-methylphenol sowie mit 1,94 Teilen 9-Decen-1-ol und 2,13 Teilen Katalysator aus Beispiel 1 versetzt. Der Ansatz wird bis zur Homogenität gerührt und anschließend vom Lösemittel durch Vakuumdestillation befreit. Der Rückstand wird 6,5 h bei 160°C unter Stickstoffatmosphäre umgesetzt. Etwaiges Kondensat wird abdestilliert oder im Grobvakuum abgezogen. Das Rohprodukt wird in Toluol aufgelöst und durch Fällung in Ethanol, Filtration und Vakuumtrocknung des Fällungsproduktes gereinigt. Die Bromzahl beträgt 2,8.

### Beispiel 3:

### Herstellung eines vinylmodifizierten Polymethylmethacrylats (VPA 2)

100 Teile trockenes Polymethylmethacrylat PA-180 (Copolymer mit geringeren Anteilen anderer Acrylate) werden in ca. 350 Teilen trockenem Toluol unter Feuchtigkeitsausschluß gelöst und diese Lösung mit 0,03-0,04 Teilen 2,6-Ditertiärbutyl-4-methylphenol sowie mit 1,55 Teilen 9-Decen-1-ol und 1,7 Teilen Katalysator aus Beispiel 1 versetzt. Der Ansatz wird bis zur Homogenität gerührt und anschließend vom Lösemittel durch Vakuumdestillation befreit. Der Rückstand wird 5,5 h bei 160°C unter Stickstoffatmosphäre umgesetzt. Etwaiges Kondensat wird abdestilliert oder im Grobvakuum abgezogen. Das Rohprodukt wird in Toluol aufgelöst und durch Fällung in Ethanol, Filtration und Vakuumtrocknung des Fällungsproduktes gereinigt. Die Bromzahl beträgt 1,6.

### Beispiel 4:

### Herstellung eines vinylmodifizierten Polymethylmethacrylats (VPA 3)

100 Teile trockenes Polymethylmethacrylat PA-30 (Copolymer mit geringeren Anteilen anderer Acrylate) werden in ca. 350 Teilen trockenem Toluol unter Feuchtigkeitsausschluß gelöst und diese Lösung mit 0,03-0,04 Teilen 2,6-Ditertiärbutyl-4-methylphenol sowie mit 1,55 Teilen 9-Decen-1-ol und 1,7 Teilen Katalysator aus Beispiel 1 versetzt. Der Ansatz wird bis zur Homogenität gerührt und anschließend vom Lösemittel durch Vakuumdestillation befreit. Der Rückstand wird 5,5 h bei 160°C unter Stickstoffatmosphäre umgesetzt. Etwaiges Kondensat wird abdestilliert oder im Grobvakuum abgezogen. Das Rohprodukt wird in Toluol aufgelöst und durch Fällung in Ethanol, Filtration und Vakuumtrocknung des Fällungsproduktes gereinigt. Die Bromzahl beträgt 2,1.

### Beispiel 5:

### Herstellung eines polysiloxanmodifizierten Polymethylmethacrylats

100 Teile trockenes vinylmodifiziertes Poly(methylmethacrylat) aus Beispiel 2 werden in ca. 2500 Teilen trockenem Toluol unter Feuchtigkeitsausschluß gelöst und anschließend mit 33,5 Teilen TMDS und 10 Teilen Pt-Katalysator-Lösung unter Rühren versetzt. Der Ansatz wird 7 h bei 65°C gerührt. Ca. 80 % des Lösemittels werden im Vakuum abdestilliert und anschließend wieder durch trockenes Toluol ersetzt. 255 Teile DVPDMS werden unter Rühren zugegeben und der Ansatz bei 70°C über einen Zeitraum von 7 h gerührt. Das Reaktionsprodukt wird in Hexan gefällt, isoliert und im Vakuum getrocknet.

### Beispiel 6:

### Herstellung eines polysiloxanmodifizierten Polymethylmethacrylats

100 Teile trockenes vinylmodifiziertes Poly(methylmethacrylat) aus Beispiel 3 werden in ca. 2500 Teilen trockenem Toluol unter Feuchtigkeitsausschluß gelöst und anschließend mit 27 Teilen TMDS und 10 Teilen Pt-Katalysator-Lösung unter Rühren versetzt. Der Ansatz wird 7 h bei 65°C gerührt. Ca. 80 % des Lösemittels werden im Vakuum abdestilliert und anschließend wieder durch trockenes Toluol ersetzt. 252 Teile VMQS werden unter Rühren zugegeben und der Ansatz bei 70°C über einen Zeitraum von 7 h gerührt. Das Reaktionsprodukt wird in Hexan gefällt, isoliert und im Vakuum getrocknet.

### Beispiel 7:

### Herstellung eines polysiloxanmodifizierten Polymethylmethacrylats

100 Teile trockenes vinylmodifiziertes Poly(methylmethacrylat) aus Beispiel 4 werden in ca. 2500 Teilen trockenem Toluol unter Feuchtigkeitsausschluß gelöst und anschließend mit 27 Teilen TMDS und 10 Teilen Pt-Katalysator-Lösung unter Rühren versetzt. Der Ansatz wird 7 h bei 65°C gerührt. Ca. 80 % des Lösemittels werden im Vakuum abdestilliert und anschließend wieder durch trockenes Toluol ersetzt. 182 Teile VMDTS werden unter Rühren zugegeben und der Ansatz bei 70°C über einen Zeitraum von 7 h gerührt. Das Reaktionsprodukt wird in Hexan gefällt, isoliert und im Vakuum getrocknet.

Die nach den Beispielen 5 bis 7 hergestellten Adhäsive werden dem nachfolgenden Testverfahren unterzogen. Das Haftvermittler-Polymer (Adhäsiv) wird zunächst in einer Konzentration von 5 Gew.% in Ethylacetat gelöst. Mit der so erhaltenen Lösung wird eine Fläche von 30 x 20 mm² eines Polyacrylat-Plättchens (Paladon 65, Fa. Kulzer) der Dimensionen 60 x 20 mm², das zuvor naß beschliffen (Körnung 500) und mit Ethanol gereinigt wurde, ausgehend von einem Ende des Plättchens einmal dünn bestrichen. Nach einer Ablüftzeit von 1 min wird auf den gesamten so präparierten Polyacrylat-Träger eine Schicht von 4 mm Dicke eines bei Raumtemperatur additionsvernetzenden Silikonmaterials (Mollosil Plus, Fa. Detax) aufgebracht. 1 h nach Erhärtung des Silikons wird die Anordnung ausgehend vom nicht anhaftenden (nicht mit Haftvermittler bestrichenen) Teil des Polyacrylat-Trägers einem 90°-Schälversuch unterzogen. Die Haftung wird qualitativ nach folgendem Schema einer Beurteilung unterzogen:
- A :: vollständiger kohäsiver Fehler des Silikons, die Haftung übersteigt die Reißfestigkeit des Silikons
- B:: adhäsiver Fehler bei spürbarer Haftung, die Haftung ist schwächer als die Reißfestigkeit des Silikons
- C:: keine Haftung

Die Ergebnisse der Versuche sind in Tabelle 2 zusammengefaßt.

| **Haftvermittler nach** | **Lagerung in Wasser (40°C)** | **Haftung** |
|---|---|---|
| Beispiel 5 | - | A |
| Beispiel 6 | - | B |
| Beispiel 7 | - | A |
| Beispiel 7 | 24 h | A |
| PA-30 (Vergleichsbeispiel) | - | C |
| VPA-3 (Vergleichsbeispiel) | - | C |

Die Ergebnisse zeigen, daß die erfindungsgemäßen Adhäsive auch unter feuchten Bedingungen eine stabile Adhäsion des Silikons am Kunststoff gewährleisten.

## Patentansprüche

1. Adhäsiv, enthaltend ein Polymer mit den folgenden Merkmalen:
- ein polymerer Backbone,
- über Esterbindungen mit dem Backbone verknüpfte Alkyl-, Aryl- und/oder Alkenylgruppen,
- über Esterbindungen mit dem Backbone verknüpfte Alkylen- und/oder Arylengruppen als Spacer,
- an den Spacer ist mittels einer Si-C-Bindung eine offenkettige und/oder cyclische Silikongruppe und/oder Silangruppe gebunden,
- an die Silikon- oder Silangruppe ist mittels einer Si-C-Bindung eine Ethylengruppe gebunden,
- die Ethylengruppe wiederum ist mit einer zweiten Silikongruppe verbunden, die M-, D-, T-, und/oder Q-Silikoneinheiten sowie wenigstens eine an Si gebundene Vinylgruppe enthält.

2. Adhäsiv nach Anspruch 1, **dadurch gekennzeichnet, daß** es folgende Strukturelemente aufweist: 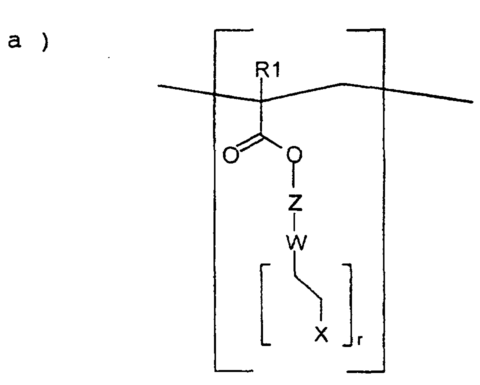 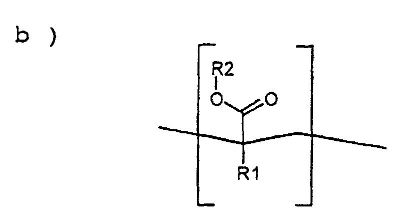 wobei bedeuten:
- r: eine ganze Zahl von 1 bis 3,
- R₁: gleich oder verschieden H, Methyl, Ethyl, Propyl oder Butyl,
- R₂: eine geradkettige oder verzweigte Alkyl-, Aryl-, Aralkyl- und/oder Alkenylgruppe,
- Z: eine geradkettige oder verzweigte Alkylen- oder Arylen- oder Aralkylengruppe,
- W: eine offenkettige und/oder cyclische Silikongruppe oder Silangruppe, die über Si-C-Bindungen verknüpft ist,
- X: eine M-, D-, T- und/oder Q-Einheiten enthaltende zweite Silikongruppe, wobei wenigstens eine Silikongruppe X wenigstens eine an Si gebundene Vinylgruppe enthält.

3. Adhäsiv nach Anspruch 2, **dadurch gekennzeichnet, daß** das Strukturelement a) wie folgt aufgebaut ist: 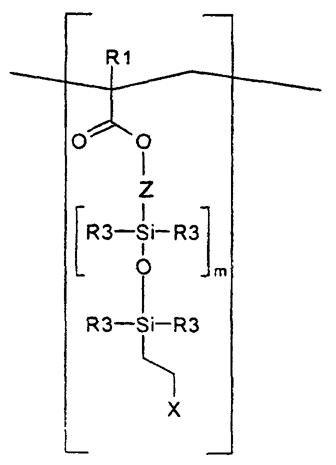 wobei R₁, R₂, Z und X die in Anspruch 2 angegebenen Bedeutungen haben, R₃ eine geradkettige oder verzweigte Alkyl- oder Aryl- oder Aralkylgruppe darstellt, und m eine ganze Zahl von 0 bis 2 ist.

4. Adhäsiv nach einem der Ansprüche 1 bis 3, **dadurch gekennzeichnet, daß** der Backbone ein Poly(meth)acrylat aufweist.

5. Adhäsiv nach Anspruch 4, **dadurch gekennzeichnet, daß** die Poly(meth)acrylatkomponente ein Molekulargewicht von 800 - 1.000.000 aufweist.

6. Adhäsiv nach einem der Ansprüche 1 bis 5, **dadurch gekennzeichnet, daß** die Silikongruppe X ein Molekulargewicht von 80 - 200.000 aufweist.

7. Adhäsiv nach einem der Ansprüche 1 bis 6, **dadurch gekennzeichnet, daß** jede Silikongruppe X mindestens eine Vinylgruppe enthält.

8. Adhäsiv nach einem der Ansprüche 1 bis 7, **dadurch gekennzeichnet, daß** der Spacer Z eine geradkettige (unverzweigte) Alkylverbindung ist.

9. Adhäsiv nach einem der Ansprüche 1 bis 8, **dadurch gekennzeichnet, daß** es zusätzlich ein nicht reaktives Lösemittel enthält.

10. Adhäsiv nach einem der Ansprüche 1 bis 9, **dadurch gekennzeichnet, daß** es zusätzlich ein oder mehrere reaktive (Meth)acrylatharze enthält.

11. Adhäsiv nach einem der Ansprüche 1 bis 10, **dadurch gekennzeichnet, daß** es zusätzlich amorphe Kieselsäure und/oder feinteilige Gläser enthält.

12. Adhäsiv nach einem der Ansprüche 1 bis 11, **dadurch gekennzeichnet, daß** es zusätzlich Pigmente enthält.

13. Verfahren zur Herstellung eines Adhäsivs gemäß einem der Ansprüche 1 bis 12, mit den Schritten:
- Umesterung eines Poly(alk)acrylats mit ethylenisch ungesättigten Alkoholen,
- Umsetzung dieser Verbindung mit flüchtigen Silanen und/oder Siloxanen, die mindestens eine und wenigstens teilweise mindestens zwei Si-H-Bindungen enthalten,
- Umsetzung des Reaktionsproduktes mit Silikonen, die mindestens eine an Si gebundene Vinylgruppe und wenigstens teilweise zwei oder mehr an Si gebundene Vinylgruppen enthalten.

14. Verfahren zur Herstellung eines Adhäsivs gemäß einem der Ansprüche 1 bis 12, mit den Schritten:
- Umsetzung eines Copolymeren aus Alkyl(meth)-acrylaten und Alkenyl(meth)acrylaten mit flüchtigen Silanen und/oder Siloxanen, die mindestens eine und wenigstens teilweise mindestens zwei Si-H-Bindungen enthalten,
- Umsetzung des Reaktionsproduktes mit Silikonen, die mindestens eine an Si gebundene Vinylgruppe und wenigstens teilweise zwei oder mehr an Si gebundene Vinylgruppen enthalten.

15. Adhäsiv gemäß einem der Ansprüche 1 bis 12 zur Verwendung als medizinisches Präparat.

16. Verwendung eines Adhäsivs gemäß einem der Ansprüche 1 bis 12 zur Herstellung eines Haftvermittlers zur Anwendung im Dentalbereich zwecks Herstellung einer kraftschlüssigen Verbindung zwischen einem Kunststoff und einem Organosiloxanmaterial.

17. Verwendung eines Adhäsivs gemäß der Ansprüche 1 bis 12 zur Herstellung eines Haftvermittlers zur Anwendung im otoplastischen Bereich zur Herstellung einer kraftschlüssigen Verbindung zwischen einem Kunststoff und einem Organopolysiloxan.

## Claims

1. Adhesive comprising a polymer having the following features:
- a polymeric backbone,
- alkyl, aryl and/or alkenyl groups attached to the backbone by way of ester linkages,
- alkylene and/or arylene spacer groups attached to the backbone by way of ester linkages,
- an open-chain and/or cyclic silicone group and/or silane group is attached to the spacer by means of an Si-C bond,
- an ethylene group is attached to the silicone and/or silane group by means of an Si-C bond,
- the ethylene group in turn is connected to a second silicone group which comprises M, D, T and/or Q silicone units and comprises at least one vinyl group attached to Si.

2. Adhesive according to Claim 1, **characterized in that** it has the following structural elements: 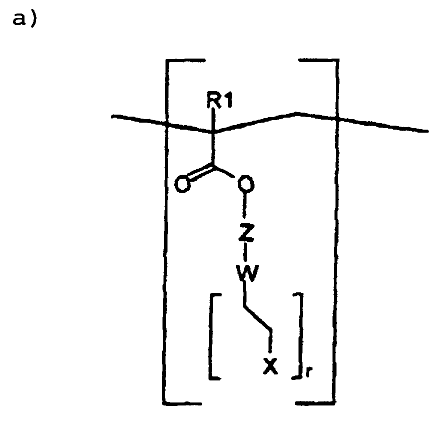 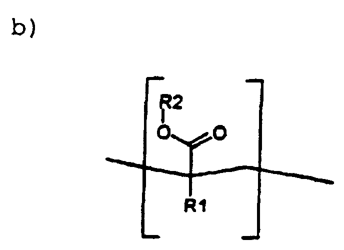 in which
- r is an integer from 1 to 3,
- R₁ is identical or different and is H, methyl, ethyl, propyl or butyl,
- R₂ is a straight-chain or branched alkyl, aryl, aralkyl and/or alkenyl group,
- Z is a straight-chain or branched alkylene or arylene or aralkylene group,
- W is an open-chain and/or cyclic silicone group or silane group which is attached by way of Si-C bonds,
- X is a second silicone group containing M, D, T and/or Q units, at least one silicone group X containing at least one vinyl group attached to Si.

3. Adhesive according to Claim 2, **characterized in that** the structural element a) has the following composition: 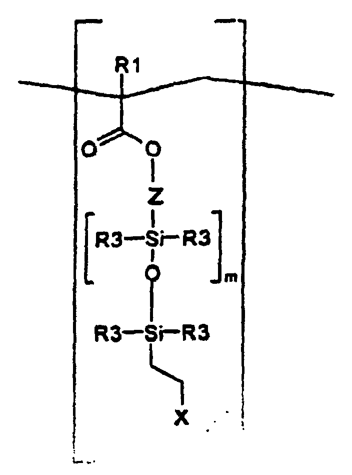 in which R₁, R₂, Z and X are as defined in Claim 2, R₃ is a straight-chain or branched alkyl or aryl or aralkyl group and m is an integer from 0 to 2.

4. Adhesive according to one of Claims 1 to 3, **characterized in that** the backbone comprises a poly(meth)acrylate.

5. Adhesive according to Claim 4, **characterized in that** the poly(meth)acrylate component has a molecular weight of 800 - 1,000,000.

6. Adhesive according to one of Claims 1 to 5, **characterized in that** the silicone group X has a molecular weight of 80 - 200,000.

7. Adhesive according to one of Claims 1 to 6, **characterized in that** each silicone group X contains at least one vinyl group.

8. Adhesive according to one of Claims 1 to 7, **characterized in that** the spacer Z is a straight-chain (unbranched) alkyl compound.

9. Adhesive according to one of Claims 1 to 8, **characterized in that** it additionally comprises a non-reactive solvent.

10. Adhesive according to one of Claims 1 to 9, **characterized in that** it additionally comprises one or more reactive (meth)acrylate resins.

11. Adhesive according to one of Claims 1 to 10, **characterized in that** it additionally comprises amorphous silica and/or finely divided glasses.

12. Adhesive according to one of Claims 1 to 11, **characterized in that** it additionally comprises pigments.

13. Process for preparing an adhesive according to one of Claims 1 to 12, comprising the steps of:
- transesterifying a poly(alk)acrylate with ethylenically unsaturated alcohols,
- reacting this compound with volatile silanes and/or siloxanes containing at least one and, at least in part, at least two Si-H bonds,
- reacting the reaction product with silicones which contain at least one Si-bonded vinyl group and, at least in part, two or more Si-bonded vinyl groups.

14. Process for preparing an adhesive according to one of Claims 1 to 12, comprising the steps of:
- reacting a copolymer of alkyl (meth)acrylates and alkenyl (meth)acrylates with volatile silanes and/or siloxanes which contain at least one and, at least in part, at least two Si-H bonds,
- reacting the reaction product with silicones which contain at least one Si-bonded vinyl group and, at least in part, two or more Si-bonded groups.

15. Adhesive according to one of Claims 1 to 12 for use as a medical preparation.

16. Use of an adhesive according to one of Claims 1 to 12 for preparing an adhesion promoter for use in the dental field for producing a frictional bond between a polymer and an organosiloxane material.

17. Use of an adhesive according to one of Claims 1 to 12 for preparing an adhesion promoter for use in the otoplastic field for producing a frictional bond between a polymer and an organopolysiloxane.

## Revendications

1. Adhésif comprenant un polymère avec les caractéristiques suivantes :
- un squelette polymère,
- des groupes alkyles, aryles et/ou alcényles réticulés avec le squelette par des liaisons esters,
- des groupes alkylènes et/ou arylènes réticulés avec le squelette par des liaisons esters et servant d'espaceurs,
- un groupe silicone et/ou silane à chaîne ouverte et/ou cyclique est lié aux espaceurs au moyen d'une liaison Si-C,
- un groupe éthylène est lié au groupe silicone ou silane au moyen d'une liaison Si-C,
- le groupe éthylène est d'autre part lié à un second groupe silicone contenant les unités silicone M, D, T et/ou Q ainsi qu'au moins un groupe vinyle lié à Si.

2. Adhésif selon la revendication 1, **caractérisé en ce qu'**il présente les éléments structurels suivants: 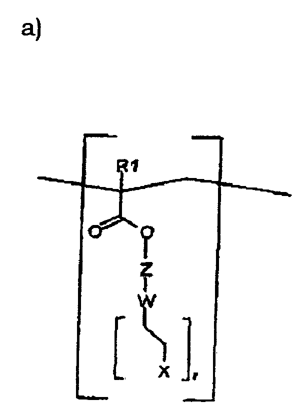 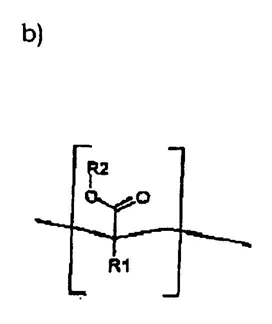 où
- r représente un nombre entier allant de 1 à 3,
- R₁ représente des H, méthyle, éthyle, propyle ou butyle identiques ou différents,
- R₂ représente un groupe alkyle, aryle, aralkyle et/ou alcényle linéaire ou ramifié,
- Z représente un groupe alkylène ou arylène ou aralkylène linéaire ou ramifié,
- W représente un groupe silicone ou silane à chaîne ouverte et/ou cyclique, réticulé par des liaisons Si-C,
- X représente un second groupe silicone contenant des unités M, D, T et/ou Q, au moins un groupe silicone X contenant au moins un groupe vinyle lié à Si.

3. Adhésif selon la revendication 2, **caractérisé en ce que** l'élément structurel a) est structuré comme suit : 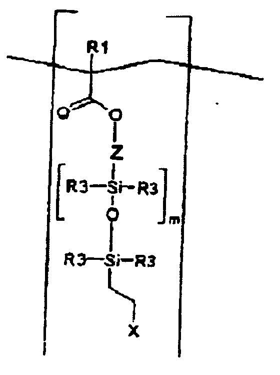 où R₁, R₂, Z et X ont la signification indiquée à la revendication 2, R₃ représente un groupe alkyle, aryle ou aralkyle linéaire ou ramifié et où m est un nombre entier allant de 0 à 2.

4. Adhésif selon l'une quelconque des revendications 1 à 3, **caractérisé en ce que** le squelette présente un poly(méth)acrylate.

5. Adhésif selon la revendication 4, **caractérisé en ce que** le composant poly(méth)acrylate présente une masse moléculaire allant de 800 à 1 000 000.

6. Adhésif selon l'une quelconque des revendications 1 à 5, **caractérisé en ce que** le groupe silicone X présente une masse moléculaire allant de 80 à 200 000.

7. Adhésif selon l'une quelconque des revendications 1 à 6, **caractérisé en ce que** chaque groupe silicone X contient au moins un groupe vinyle.

8. Adhésif selon l'une quelconque des revendications 1 à 7, **caractérisé en ce que** l'espaceur Z est un composé alkyle linéaire (non ramifié).

9. Adhésif selon l'une quelconque des revendications 1 à 8, **caractérisé en ce qu'**il contient en plus un solvant non réactif.

10. Adhésif selon l'une quelconque des revendications 1 à 9, **caractérisé en ce qu'**il contient en plus une ou plusieurs résines (méth)acrylates réactives.

11. Adhésif selon l'une quelconque des revendications 1 à 10, **caractérisé en ce qu'**il contient en plus de l'acide silicique amorphe et/ou des verres finement divisés.

12. Adhésif selon l'une quelconque des revendications 1 à 11, **caractérisé en ce qu'**il contient en plus des pigments.

13. Procédé de fabrication d'un adhésif selon l'une quelconque des revendications 1 à 12, comprenant les étapes de :
- transestérification d'un poly(alkyl)acrylate avec des alcools à insaturation éthylénique,
- réaction de ce composé avec des silanes et/ou siloxanes volatils qui contiennent au moins une et au minimum en partie au moins deux liaisons Si-H,
- transformation du produit de la réaction avec des silicones qui contiennent au moins un et au minimum en partie deux ou plusieurs groupes vinyles liés à Si.

14. Procédé de fabrication d'un adhésif selon l'une quelconque des revendications 1 à 12, comprenant les étapes de :
- réaction d'un copolymère de (méth)acrylates d'alkyle et de (méth)acrylates d'alcényle avec des silanes et/ou siloxanes volatils qui contiennent au moins une et au minimum en partie au moins deux liaisons Si-H,
- transformation du produit de la réaction avec des silicones qui contiennent au moins un et au minimum en partie deux ou plusieurs, groupes vinyles liés à Si.

15. Adhésif selon l'une quelconque des revendications 1 à 12 destiné à être utilisé comme préparation médicale.

16. Utilisation d'un adhésif selon l'une quelconque des revendications 1 à 12 pour la fabrication d'un agent adhésif pour une application dans le domaine dentaire en vue de produire une adhérence entre une matière plastique et un matériau organosiloxane.

17. Utilisation d'un adhésif selon l'une quelconque des revendications 1 à 12 pour la fabrication d'un agent adhésif pour une application dans le domaine de l'otoplastie en vue de produire une adhérence entre une matière plastique et un organopolysiloxane.
